# EUROPEAN PATENT SPECIFICATION

(11) **EP 2 617 361 B1**
(45) Date of publication and mention of the grant of the patent: **14.05.2014**
(21) Application number: 12802773.7
(22) Date of filing: 19.04.2012
(51) Int. Cl.: A61B 8/12, A61B 10/02

(54) **BIOPSY TREATMENT TOOL**
BIOPSIE-BEHANDLUNGSWERKZEUG
OUTIL DE TRAITEMENT DE BIOPSIE

(30) Priority: 23.06.2011 JP 2011139787
(43) Date of publication of application: 24.07.2013
(73) Proprietor: OLYMPUS MEDICAL SYSTEMS CORP., Tokyo 151-0072 (JP)
(72) Inventor: NISHINA, Kenichi, Tokyo 151-0072 (JP); OKUNO, Yoshiyuki, Tokyo 151-0072 (JP); KOMURO, Masahiko, Tokyo 151-0072 (JP); IMAHASHI, Takuya, Tokyo 151-0072 (JP); MIYAKI, Hironaka, Tokyo 151-0072 (JP)
(74) Representative: Gunzelmann, Rainer
(86) International application number: PCT/JP2012/060587
(87) International publication number: WO 2012/176543

(56) References cited:
- JP-A- 11 206 759
- JP-A- H11 206 759
- JP-A- 2001 104 316
- JP-A- 2010 274 123
- US-A- 5 383 460
- US-A1- 2002 022 788
- US-A1- 2010 280 316
- US-A1- 2011 066 073

## Description

### Technical Field

The present invention relates to a biopsy treatment instrument inserted into a body of a subject to sample a specimen of a target region under ultrasound observation.

### Background Art

Conventionally, a biopsy is known as a method of diagnosing a lesion portion or the like in a body. When the biopsy is performed, firstly, after a target region where the lesion portion or the like is present is specified, a specimen of the target region is sampled and subjected to the biopsy.

The specification of the target region is performed using, for example, an ultrasound probe disclosed in Japanese Patent Application Laid-Open Publication No. 2004-216159 (hereinafter referred to as a "first literature"). That is, the ultrasound probe is inserted into the body and the target region is specified on the basis of an ultrasound signal received by an ultrasound transducer provided at a distal end portion of the ultrasound probe. In this case, for example, the ultrasound probe is attached to a tube-like sheath, both of the ultrasound probe and the tube-like sheath are inserted into the body, and after the target region is specified by the ultrasound probe, the ultrasound probe is removed in a state in which the tube-like sheath is left indwelling in the region.

Subsequently, a biopsy treatment instrument such as a biopsy forceps or a brush is inserted into the tube-like sheath, a distal end portion of the biopsy treatment instrument is led to the target region along the tube-like sheath, and a specimen such as a lesion tissue or a cell of the target region is sampled. In this case, it is checked under an X-ray observation whether or not a distal end of the biopsy treatment instrument reaches the target region.

As the biopsy treatment instrument, for example, a biopsy treatment instrument disclosed in Japanese Patent Application Laid-Open Publication No. 2001-104316 (hereinafter referred to as a "second literature") is known. The biopsy treatment instrument disclosed in the second literature includes a needle shaft having a sharp needle tip at a distal end and a sheath sheathed over the needle shaft to freely advance and retract. A recessed portion for sampling a specimen is formed on a side surface on a distal end side of the needle shaft. An annular edge for cutting the specimen stored in the recessed portion is formed at a distal end of the sheath.

In a technique disclosed in the second literature, the biopsy treatment instrument is stuck into a subject, after the distal end is caused to reach the target region, the sheath is pulled back a little to open the recessed portion and the specimen is stored in the recessed portion. Subsequently, the sheath is pushed forward, the specimen captured into the recessed portion is cut by the annular edge at the distal end of the sheath, and the recessed portion is closed by the sheath to store the specimen in the recessed portion.

However, in the techniques disclosed in the above-described first literature and second literature, a procedure for specifying a target region using the ultrasound probe and a procedure for sampling a specimen of the target region using the biopsy treatment instrument are performed separately.

In addition, it is impossible to determine whether or not the specimen is indwelled in the recessed portion formed at the distal end of the biopsy treatment instrument until the biopsy treatment instrument is removed from the tube-like sheath and visual check is performed. When the specimen is not indwelled in the recessed portion, it is necessary to insert the distal end of the biopsy treatment instrument to the target region again to sample the specimen.

In view of the above-described circumstances, an object of the present invention is to provide a biopsy treatment instrument capable of surely sampling a specimen of a target region such as a lesion portion by one insertion, and capable of reducing a burden on a subject.

US 5 383 460 A discloses a catheter and imagine system. The catheter comprises a rigid housing at the distal end of the catheter, a cutter attached to a drive cable and rotatably and axially slidably positioned within the housing, an ultrasonic transducer attached to the cutter and a flexible nose section.

US 2011/066073 A1 relates to a biopsy device, particularly a biopsy device comprising a shaft with a transducer element for providing information about acoustic properties of a material to be analysed, a system of positioning a biopsy device and a method for positioning a biopsy device. The biopsy device may be adapted to take biopsies of different regions of the human body for excluding or detecting abnormalities as cancerous lesions. The biopsy device may be used to measure acoustic properties of the material while inserting the tip portion of the biopsy device into the material to be analysed. The biopsy device may further allow measurement based on elastography.

### Disclosure of Invention

### Means for Solving the Problem

A biopsy treatment instrument according to claim 1 is provided.

### Brief Description of the Drawings

Fig. 1 is a perspective view of a biopsy treatment instrument according to a first embodiment;
Fig. 2 is a partial sectional side view of a distal end portion of the biopsy treatment instrument according to the first embodiment;
Fig. 3 is a sectional side view of Fig. 2 according to the first embodiment;
Fig. 4 is a IV-IV sectional view of Fig. 3 according to the first embodiment;
Fig. 5(a) is a waveform chart of a wave reception signal of a c-MUT according to the first embodiment, and Fig. 5(b) is a waveform chart of a wave reception signal after waveform shaping according to the first embodiment;
Fig. 6 is a sectional view equivalent to Fig. 2 showing a state in which a specimen is captured into a recessed portion for specimen sampling according to the first embodiment;
Fig. 7 is a sectional view equivalent to Fig. 6 showing a state in which the specimen captured into the recessed portion for specimen sampling is being cut according to the first embodiment;
Fig. 8 is a sectional view equivalent to Fig. 7 showing a state in which the specimen captured into the recessed portion for specimen sampling is cut according to the first embodiment;
Figs. 9(a), 9(b) and 9(c) show wave reception waveforms of the C-MUT arranged in the recessed portion for specimen sampling according to the first embodiment, wherein Fig. 9(a) is a waveform chart of a state in which the recessed portion for specimen sampling is filled with air, Fig. 9(b) is a waveform chart of a state in which the recessed portion for specimen sampling is filled with liquid, and Fig. 9(c) is a waveform chart of a state in which the specimen is indwelled in the recessed portion for specimen sampling;
Fig. 10 is a sectional view equivalent to Fig. 3 according to a second embodiment;
Fig. 11 is a XI-XI sectional view of Fig. 10 according to the second embodiment;
Fig. 12 is a sectional view equivalent to Fig. 6 according to the second embodiment;
Fig. 13 is a sectional view equivalent to Fig. 8 according to the second embodiment;
Fig. 14 is a sectional view equivalent to Fig. 3 according to a third embodiment;
Fig. 15 is a sectional view equivalent to Fig. 3 according to a fourth embodiment;
Fig. 16 is a sectional view equivalent to Fig. 3 according to a fifth embodiment;
Fig. 17 is a XVII-XVII sectional view of Fig. 16 according to the fifth embodiment;
Fig. 18 is a sectional view equivalent to Fig. 17 according to another aspect of the fifth embodiment;
Fig. 19 is a sectional view equivalent to Fig. 17 according to yet another aspect of the fifth embodiment;
Fig. 20 is a sectional view equivalent to Fig. 17 according to yet another aspect of the fifth embodiment;
Fig. 21 is a configuration diagram of an endoscope system including a biopsy treatment instrument according to a sixth embodiment; and
Fig. 22 is a functional configuration diagram of the endoscope system according to the sixth embodiment.

### Best Mode for Carrying Out the Invention

Embodiments of the present invention are explained below with reference to the drawings. Note that the drawings are schematic and relations among thicknesses and widths of respective members, ratios of the thicknesses of the respective members, and the like are different from real ones. It goes without saying that portions where relations and ratios of dimensions are different from one another are included among the drawings.

### [First Embodiment]

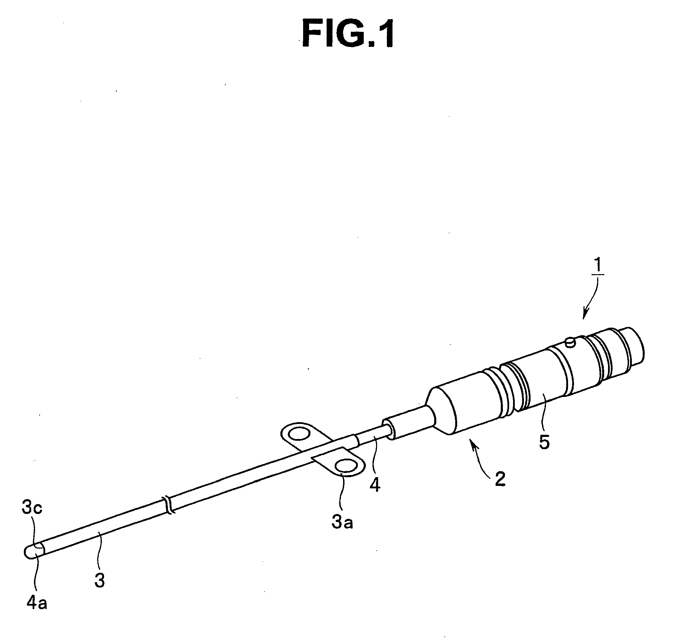

A first embodiment of the present invention is shown in Figs. 1 to 9. Reference numeral 1 in Fig. 1 denotes a biopsy treatment instrument. The biopsy treatment instrument 1 includes a treatment instrument main body 2 and a flexible sheath 3 functioning as a tubular portion. Further, the treatment instrument main body 2 includes an insertion portion 4 functioning as an elongated and thin bar-like portion having flexibility. A connector portion 5 is provided at a proximal end portion of the insertion portion 4. Note that the connector portion 5 is connected to a not-shown ultrasound observation apparatus.

The insertion portion 4 is inserted into the flexible sheath 3 in a state in which the insertion portion 4 is capable of advancing and retracting relative to the flexible sheath 3. A slide portion 6 that reduces frictional resistance in relatively moving the flexible sheath 3 and the insertion portion 4 in an axis direction is provided between an inner circumference of the flexible sheath 3 and an outer circumference of the insertion portion 4. Note that, in the present embodiment, a low friction coefficient layer is formed on one or both of an inner circumferential surface of the flexible sheath 3 and an outer circumferential surface of the insertion portion 4 using a material having no effect on a human body. The low friction coefficient layer is formed as the slide portion 6.

The relative slide in this context may be any one of fixing the insertion portion 4 and sliding the slide portion 6 back and forth, fixing the slide portion 6 and sliding the insertion portion 4 back and forth, and sliding both of the insertion portion 4 and the slide portion 6.

When the flexible sheath 3 and the insertion portion 4 are formed of a material having high slippage, for example, fluorine resin such as polytetrafluoroethylene (PTFE) or polyethylene, an appropriate clearance may only be provided to form the slide portion 6 without forming the low friction coefficient layer.

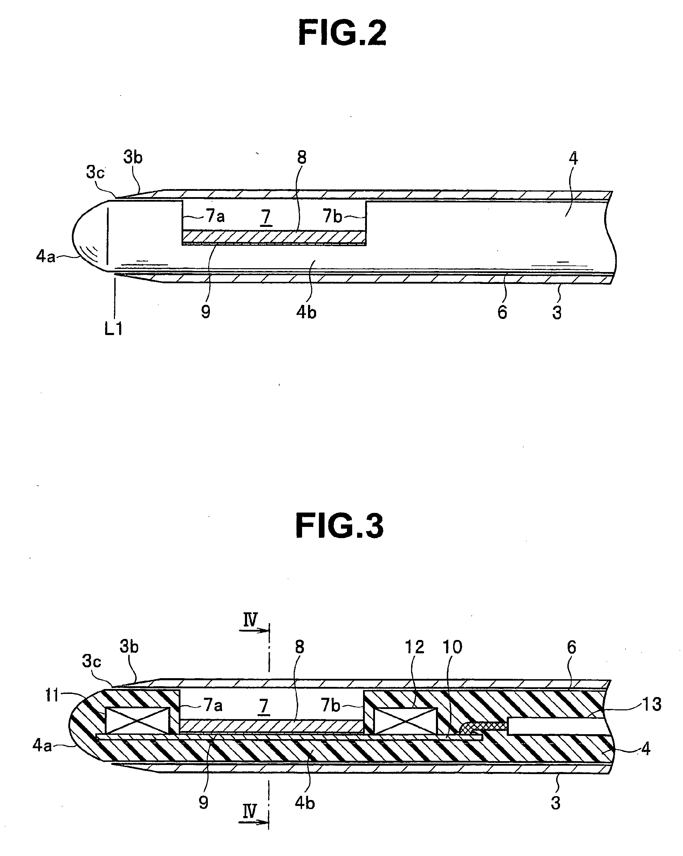

The insertion portion 4 is an elongated and thin solid shaft having flexibility. As shown in Fig. 2, a distal end face 4a is formed in a rotating body shape of a conical curve centering on an axis of the insertion portion 4. As the rotating body shape of the conical curve, there are an oval shape, a bullet shape, a semispherical shape, and the like. Note that an X-ray non-transmitting marker (not shown) that can be displayed on an X-ray fluoroscopic image or a CT image is disposed on the distal end face 4a.

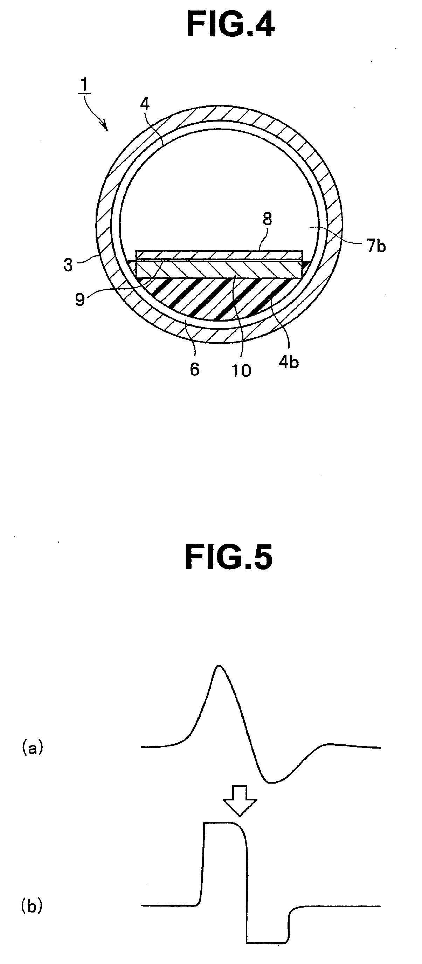

Further, a recessed portion for specimen sampling 7 functioning as a recessed portion is formed at a distal end portion of the insertion portion 4 and at a rear of the distal end face 4a. The recessed portion for specimen sampling 7 is formed by cutting out the insertion portion 4. The recessed portion for specimen sampling 7 is formed in a semi-cylindrical sectional shape, i.e., the recessed portion for specimen sampling 7 includes wall surfaces 7a and 7b formed in front and back in the axis direction and is perforated in a side surface direction. As shown in Fig. 4, a region of the insertion portion 4 left after the recessed portion for specimen sampling 7 is formed is a base portion 4b having a flat convex shape in section.

On the other hand, a sheath operation portion 3 a formed in a finger-placing shape is formed at a proximal end portion of the flexible sheath 3. An operator places a finger on the sheath operation portion 3 a to perform operations including advancing, retracting and rotating the flexible sheath 3 along the outer circumference of the insertion portion 4. A distal end outer surface 3b of the flexible sheath 3 is formed on a single-edge shaped inclined surface converging in a distal end direction. A distal end edge 3c is formed at a distal end of the sheath 3.

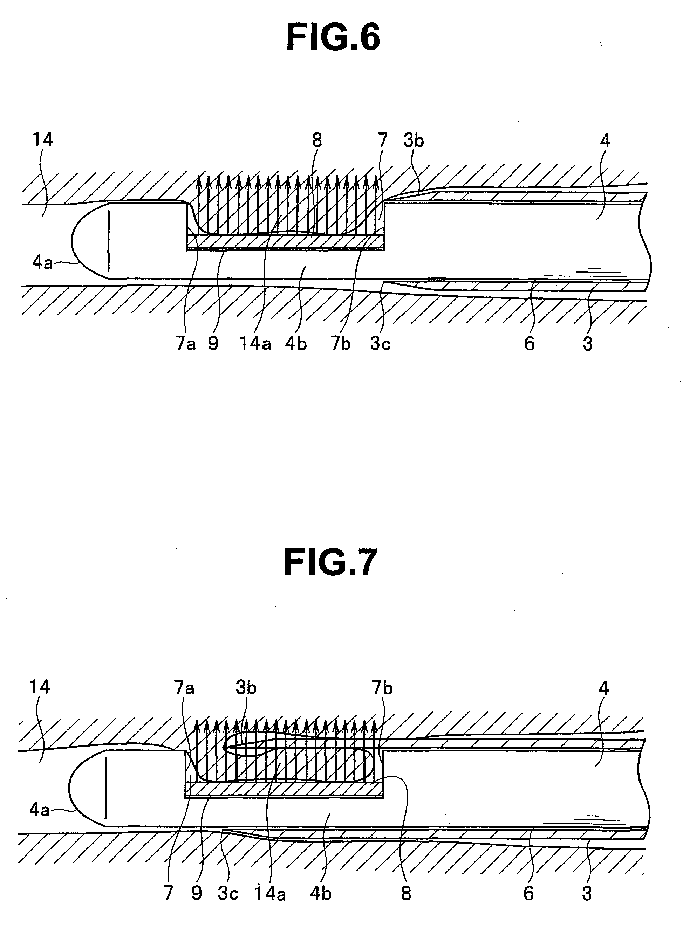

Movement amounts in an advancing and retracting direction of the flexible sheath 3 and the insertion portion 4 are regulated by a not-shown movement regulating portion in a state in which a movement in a rotating direction is allowed. That is, as shown in Fig. 2, a maximum extruded position L1 of the flexible sheath 3 is set in a position where the distal end edge 3c is continued to a rear end of the distal end face 4a of the insertion portion 4. In this state, the recessed portion for specimen sampling 7 is closed by the flexible sheath 3. On the other hand, as shown in Fig. 6, a maximum retracted position of the flexible sheath 3 is set in a position where the distal end edge 3c is at the same level as or slightly retracted from an upper end of the rear portion wall surface 7b of the recessed portion for specimen sampling 7.

The distal end outer surface 3b of the flexible sheath 3 is set to be an inclined surface extending in a tangential direction of the distal end face 4a of the insertion portion 4 in the maximum extruded position L1 shown in Fig. 2. Note that a space of about several micrometers is set between the outer circumferential surface of the insertion portion 4 and the inner circumferential surface of the flexible sheath 3.

Therefore, in a state in which the distal end edge 3c of the flexible sheath 3 is faced the maximum extruded position L1, since the distal end outer surface 3b of the flexible sheath 3 is arranged on a tangent of the distal end face 4a of the insertion portion 4, a step that scratches an inner wall in a body does not occur in a boundary between the flexible sheath 3 and the insertion portion 4. Similarly, in the figure, thickness of the slide portion 6 is illustrated to be relatively large for convenience of explanation. However, actually, the thickness is about several micrometers. A step is not caused by the slide portion 6.

A capacitive ultrasound transducer array 8 functioning as an ultrasound observation section is arranged on a bottom surface of the recessed portion for specimen sampling 7 formed in the insertion portion 4, in other words, on the base portion 4b. The capacitive ultrasound transducer array 8 is, for example, a c-MUT (capacitive-micro-machined ultrasonic transducers) manufactured using a micromachine manufacturing process. For example, an ultrasound image by linear electron scanning can be obtained by the capacitive ultrasound transducer array 8. A piezoelectric ultrasound transducer can also be used as the ultrasound observation section.

As shown in Figs. 3 and 4, a bottom surface of the capacitive ultrasound transducer array 8 is mounted on a flexible printed board (FPC: flexible printed circuits) 10 via a backing material 9 that absorbs propagation of ultrasound. The flexible printed board 10 is insert-molded in the insertion portion 4. A front portion of the flexible printed board 10 is extended into the distal end face 4a of the insertion portion 4. A rear portion of the flexible printed board 10 is extended to a rear of the recessed portion for specimen sampling 7. Further, in regions of the flexible printed board 10 extended to the distal end face 4a and the rear of the recessed portion for specimen sampling 7, two integrated circuit elements (ICs) 11 and 12 are respectively mounted as circuit elements that process an ultrasound signal transmitted and received by the capacitive ultrasound transducer array 8. Further, one end of a lead wire 13 is electrically connected to the flexible printed board 10. The other end of the lead wire 13 is extended to the connector portion 5 provided on the proximal end side through the inside of the insertion portion 4.

The connector portion 5 is connected to the not-shown ultrasound observation apparatus, whereby supply of electric power from the ultrasound observation apparatus to the flexible printed board 10 side and transfer of signals between the flexible printed board 10 and the ultrasound observation apparatus are performed.

In this case, one of the integrated circuit elements 11 and 12 is caused to function as a waveform shaping circuit that subjects an ultrasound driving signal applied to the capacitive ultrasound transducer array 8 to waveform shaping or function as an amplifying circuit that amplifies a received ultrasound signal. The other of the integrated circuit elements 11 and 12 is caused to function as a multiplexer that sequentially switches a plurality of transducers included in the capacitive ultrasound transducer array 8.

When one of the integrated circuit elements 11 and 12 is caused to function as the waveform shaping circuit, the waveform shaping circuit subjects an ultrasound driving signal applied to the respective transducers of the capacitive ultrasound transducer array 8 shown in Fig. 5(a) to rectangular processing in a predetermined manner and outputs the ultrasound driving signal, for example, as shown in Fig. 5(b). When the other of the integrated circuit elements 11 and 12 is caused to function as a multiplexer, it is possible to further reduce a line diameter of the lead wire 13 and insertion into an extremely narrow body interior such as a lung periphery is enabled. Note that reference numeral 14 in Fig. 6 denotes a body interior of a subject and reference numeral 14a denotes a specimen cut from a target region of the body interior 14. Arrows in Figs. 6 and 7 indicate an emitting direction of ultrasound.

An aspect of use of the biopsy treatment instrument 1 having such a configuration is explained. Firstly, a surgeon connects the connector portion 5 provided on a proximal end side of the treatment instrument main body 2 to the ultrasound observation apparatus.

When the connector portion 5 of the biopsy treatment instrument 1 is connected to the ultrasound observation apparatus, an ultrasound driving signal is outputted from the ultrasound observation apparatus. The ultrasound driving signal is inputted to the multiplexer provided in the other of the integrated circuit elements 11 and 12 via the lead wire 13. The multiplexer receives the ultrasound driving signal, sequentially drives the respective transducers of the capacitive ultrasound transducer array 8 fixedly provided on the bottom surface of the recessed portion for specimen sampling 7, and performs ultrasound scanning using ultrasound emitted from the respective transducers.

Subsequently, the insertion portion 4 of the treatment instrument main body 2 and the flexible sheath 3 sheathed over the insertion portion 4 are inserted into the body interior 14. In the insertion, as shown in Fig. 2, since the distal end outer surface 3b of the flexible sheath 3 is in a state of being continued in the tangential direction with respect to the distal end face 4a of the insertion portion 4, a step does not occur in a boundary between the distal end face 4a and the distal end edge 3c. An inner wall of the body interior 14 is not scratched in the insertion. As shown in Fig. 3, since the integrated circuit element 11 is insert-molded in the distal end face 4a, the distal end portion is hardened, guidability in the body interior 14 by the distal end face 4a is improved. Further, manufacturing is facilitated because it is unnecessary to change a material of the distal end face 4a at all.

Incidentally, since the X-ray non-transmitting marker (not shown) is provided on the distal end face 4a of the insertion portion 4, it is possible to check as appropriate, using an X-ray fluoroscopic image or a CT image, to where the distal end of the insertion portion 4 is inserted. Note that the capacitive ultrasound transducer array 8 and the ultrasound observation apparatus are electrically connected via the multiplexer provided in the other of the integrated circuit elements 11 and 12. Therefore, the line diameter of the lead wire 13 can be further reduced and a line diameter of the insertion portion 4 can also be reduced by a corresponding amount. Therefore, it is possible to insert the insertion portion 4 into an extremely narrow body interior of a lung peripheral or the like.

When the distal end face 4a of the insertion portion 4 reaches a target region, the surgeon operates the sheath operation portion 3a formed on the side of the surgeon's hand of the flexible sheath 3, pulls the flexible sheath 3 to the surgeon's side, and, as shown in Fig. 6, moves the distal end edge 3c of the flexible sheath 3 further backward than the rear portion wall surface 7b of the recessed portion for specimen sampling 7 formed in the insertion portion 4 to open the recessed portion for specimen sampling 7.

When the recessed portion for specimen sampling 7 is closed by the flexible sheath 3, most of reflected waves received by the capacitive ultrasound transducer array 8 are reflected waves from an inner surface of the flexible sheath 3. Therefore, waveforms of the reflected waves are substantially constant. On the other hand, when the flexible sheath 3 is pulled to open the recessed portion for specimen sampling 7, ultrasound emitted from the capacitive ultrasound transducer array 8 is reflected by a living body tissue of the body interior 14 and received. Therefore, a reflected wave of the ultrasound has a different waveform. Therefore, the surgeon can recognize, by visually recognizing the waveform with a monitor or the like, whether or not the recessed portion for specimen sampling 7 is opened.

The target region of the body interior 14 is sometimes constricted by a lesion portion or the like. Therefore, the distal end face 4a of the insertion portion 4 is inserted by expanding the constricted portion. Therefore, when the flexible sheath 3 is retracted to open the recessed portion for specimen sampling 7, a region to be the specimen 14a such as the lesion portion is captured into the recessed portion for specimen sampling 7. When the region to be the specimen 14a is captured into the recessed portion for specimen sampling 7, a distance between the capacitive ultrasound transducer array 8 and the region to be the specimen 14a decreases. Therefore, a period until the ultrasound emitted from the capacitive ultrasound transducer array 8 is reflected from a cell tissue of the region to be the specimen 14a and received decreases.

Therefore, the surgeon can grasp, by checking a change in the waveform of the reflected wave with the monitor or the like, whether or not the region to be the specimen 14a is captured into the recessed portion for specimen sampling 7. Note that, when a large change is not observed in the waveform of the reflected wave displayed on the monitor or the like when the recessed portion for specimen sampling 7 is opened, the surgeon determines that the region to be the specimen 14a is not captured into the recessed portion for specimen sampling 7, moves the insertion portion 4 in a front-back direction or rotates the insertion portion 4 to the left and right and causes the recessed portion for specimen sampling 7 to capture the region to be the specimen 14a.

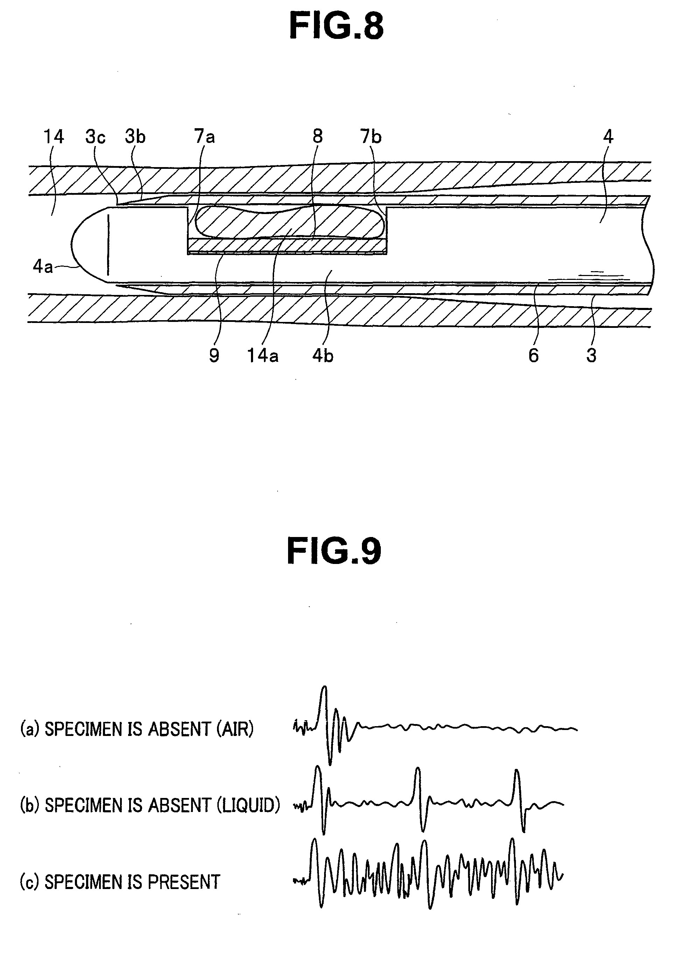

After checking that the region to be the specimen 14a is captured into the recessed portion for specimen sampling 7, the surgeon pushes the flexible sheath 3 forward while placing a finger on the sheath operation portion 3 a on the side of the surgeon's hand and rotating the sheath operation portion 3a to the left and right. Then, as shown in Fig. 7, the specimen 14a is cut by the distal end edge 3c and a portion to be excised is held between the distal end edge 3c and an upper end of the front portion wall surface 7a of the recessed portion for specimen sampling 7 and gradually pressed and cut. Thereafter, as shown in Fig. 8, when the distal end edge 3c passes the recessed portion for specimen sampling 7, the specimen 14a is completely cut off from the target region and captured into the recessed portion for specimen sampling 7. In addition, the recessed portion for specimen sampling 7 is closed by the flexible sheath 3.

Incidentally, when the flexible sheath 3 is extruded to attempt to cut the specimen 14a, the region to be the specimen 14a sometimes slips through the distal end edge 3c without being cut in. When the region to be the specimen 14a slips through the distal end edge 3c without being cut in, the region to be the specimen 14a escapes to the body interior 14 side as the distal end edge 3c approaches the front portion wall surface 7a of the recessed portion for specimen sampling 7 and a space (between the distal end edge 3c and the front portion wall surface 7a) narrows. As a result, the surgeon fails in sampling the specimen 14a.

In the present embodiment, the surgeon grasps on the basis of the waveform of the reflected wave received by the capacitive ultrasound transducer array 8 whether or not the region to be the specimen 14a is captured into the recessed portion for specimen sampling 7. Therefore, it is possible to prevent the surgeon from removing the biopsy treatment instrument 1 without sampling the specimen 14a. Differences among waveforms of the reflected wave in the case of a failure in the capturing of the specimen 14a into the recessed portion for specimen sampling 7 and the case of a success in the capturing are shown in Fig. 9. Fig. 9(a) is a waveform obtained when the recessed portion for specimen sampling 7 is filled with air, i.e., in the case of a failure in capturing the specimen 14a. When the recessed portion for specimen sampling 7 is filled with the air, since ultrasound propagation speed of the air is low and the ultrasound is not reflected, ultrasound signals other than a first ultrasound signal are hardly received by the capacitive ultrasound transducer array 8. Similarly, in Fig. 9(b), when the recessed portion for specimen sampling 7 is filled with liquid, since ultrasound propagation speed of the liquid is high compared with the air, the reflected wave from the inner wall surface of the flexible sheath 3 is received. Therefore, when the waveforms shown in Figs. 9(a) and 9(b) are detected, the surgeon determines that capturing the specimen 14a is failed.

On the other hand, as shown in Fig. 9(c), when the specimen 14a is captured into the recessed portion for specimen sampling 7, the ultrasound from the capacitive ultrasound transducer array 8 is reflected on the cell tissue of the specimen 14a approaching the capacitive ultrasound transducer array 8. Therefore, a large number of reflected waves are received by the capacitive ultrasound transducer array 8.

Therefore, when the recessed portion for specimen sampling 7 is closed by the flexible sheath 3, the reflected wave received by the capacitive ultrasound transducer array 8 is detected. When a reflected waveform shown in Fig. 9(c) is detected, the surgeon determines that capturing the specimen 14a is succeeded.

On the other hand, when reflected waveforms shown in Figs. 9(a) and 9(b) are detected, the surgeon determines that capturing the specimen 14a is failed. The surgeon pulls the flexible sheath 3 again to open the recessed portion for specimen sampling 7 and move the insertion portion 4 in the front-back direction and rotate the insertion portion 4 to the left and right. The surgeon determines from a reflected waveform displayed on the monitor or the like whether the region to be the specimen 14a is captured. When it is confirmed that the region to be the specimen 14a is captured, the surgeon moves the flexible sheath 3 forward while rotating the flexible sheath 3, cuts the specimen with the distal end edge 3c, and indwells the specimen in the recessed portion for specimen sampling 7.

As explained above, in the present embodiment, in sampling the specimen 14a in the target region in the body interior 14 using the recessed portion for specimen sampling 7, the surgeon determines from the waveform of the reflected wave received by the capacitive ultrasound transducer array 8 fixedly provided in a bottom portion of the recessed portion for specimen sampling 7 whether capturing the specimen 14a is succeeded or failed. Therefore, it is unnecessary to remove the insertion portion 4 from the body interior 14 every time and visually check whether or not the specimen 14a is captured into the recessed portion for specimen sampling 7. The surgeon can surely succeed in the sampling of the specimen 14a by inserting the insertion portion 4 once. Therefore, it is possible to efficiently sample the specimen 14a, and thus, it is possible to reduce a burden on the subject.

Note that, as the waveform of the reflected wave displayed on the monitor or the like, waveform lines shown in Fig. 9 may be directly displayed. However, the surgeon may determine, by integrating an absolute value of the waveform and displaying an integrated value as a numerical value [%] or the like, to which degree the specimen 14a can be captured. Further, in this case, a threshold indicating whether the surgeon succeeds or fails in sampling may also be displayed. Alternatively, B mode display for converting height of the waveform into luminance and displaying the luminance may be performed.

### [Second Embodiment]

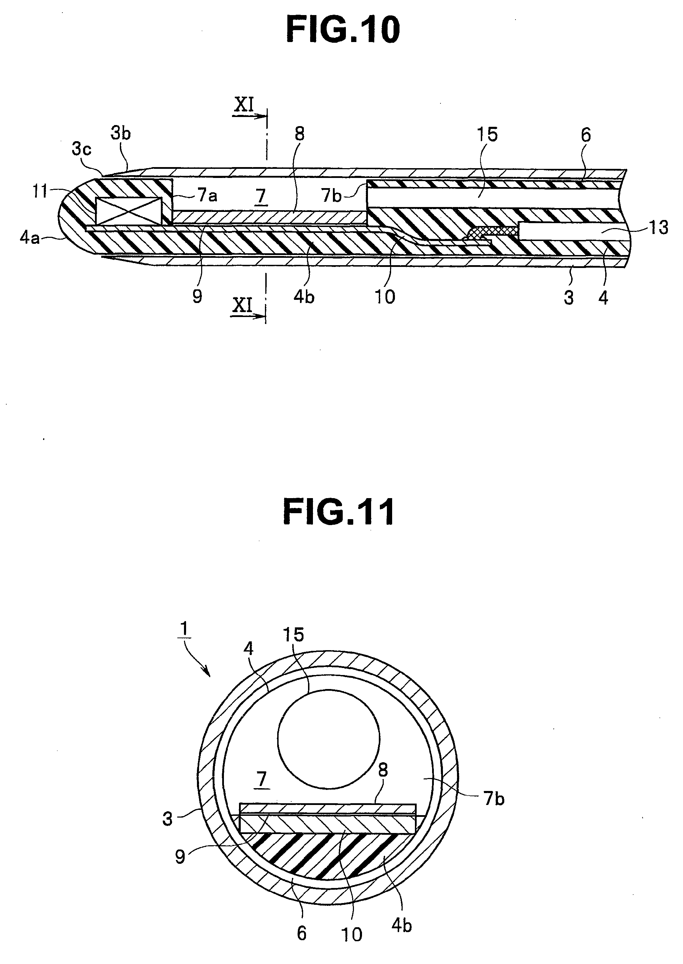

A second embodiment of the present invention is shown in Figs. 10 to 13. Note that components the same as the components in the first embodiment are denoted by the same reference numerals and signs and explanation of the components is omitted. In the present embodiment, a suction port of a suction conduit 15 is opened in the recessed portion for specimen sampling 7. The region to be the specimen 14a captured into the recessed portion for specimen sampling 7 is sucked by the suction port of the suction conduit 15 to surely indwell the region to the specimen 14a in the recessed portion for specimen sampling 7.

That is, as shown in Figs. 10 and 11, the suction conduit 15 is formed along the axis direction in the insertion portion 4. One end of the suction conduit 15 is opened in the rear portion wall surface 7b of the recessed portion for specimen sampling 7. The other end of the suction conduit 15 is opened at a rear end of the connector portion 5 (see Fig. 1). On the other hand, a suction pump or a syringe for suction (not shown) is provided in an ultrasound observation apparatus (not shown). When the connector portion 5 is connected to the ultrasound observation apparatus (not shown), the suction pump or the syringe for suction and the suction conduit 15 communicate with each other.

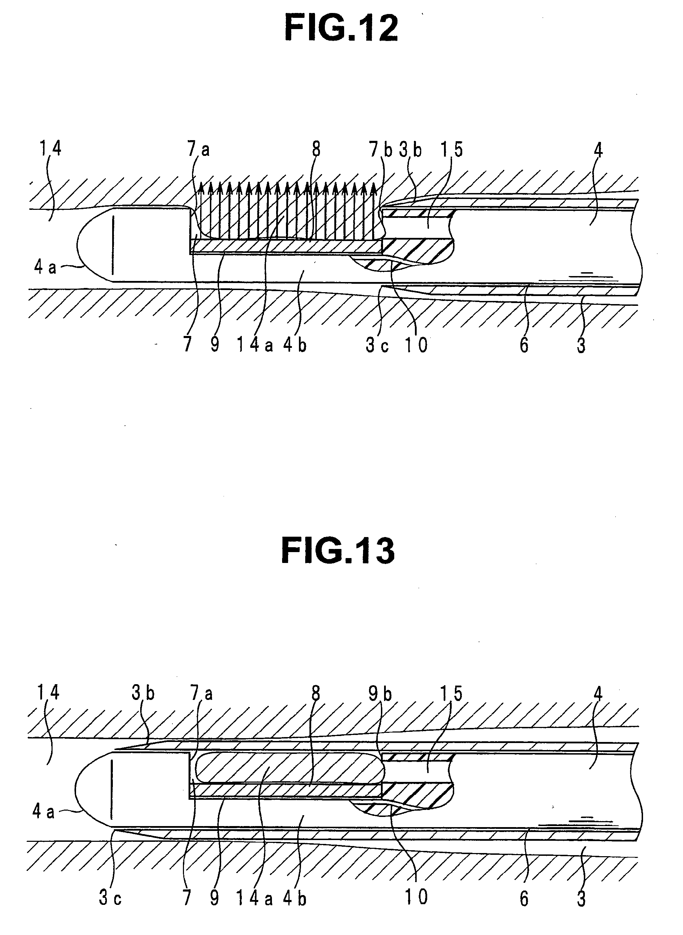

As shown in Fig. 12, when the flexible sheath 3 is retracted to open the recessed portion for specimen sampling 7, the suction pump or the syringe for suction is connected to the suction conduit 15 and a negative pressure is generated in the suction port of the suction conduit 15 opened in the rear portion wall surface 7b of the recessed portion for specimen sampling 7. Then, a part of the region to be the specimen 14a captured into the recessed portion for specimen sampling 7 is sucked to the suction port of the suction conduit 15 and held.

When the flexible sheath 3 is extruded forward in this state, as shown in Fig. 13, since the region to be the specimen 14a is sucked to the suction port of the suction conduit 15, the region to be the specimen 14a can be indwelled in the recessed portion for specimen sampling 7 without being pulled by the distal end edge 3c of the flexible sheath 3.

As a result, the specimen 14a can be surely cut by the distal end edge 3c. The slip-through of the region to be the specimen 14a in the first embodiment is reduced. It is possible to substantially reduce a frequency of failure in cutting the specimen 14a. In the present embodiment, the integrated circuit element 11 on the distal end face 4a side of the insertion portion 4 is caused to function as a multiplexer. The other integrated circuit element 12 is omitted. However, if the integrated circuit element 12 can be disposed in a region not interfering with the suction conduit 15, it is unnecessary to omit the integrated circuit element 12.

As explained above, according to the present embodiment, a part of the region to be the specimen 14a captured into the recessed portion for specimen sampling 7 is sucked to the suction port of the suction conduit 15. Therefore, when the region to be the specimen 14a is cut by the distal end edge 3c of the flexible sheath 3, the region to be the specimen 14a can be surely indwelled in the recessed portion for specimen sampling 7. As a result, it is possible to more efficiently sample the specimen 14a, realize a reduction in a sampling time period, and further reduce a burden on a subject.

### [Third Embodiment]

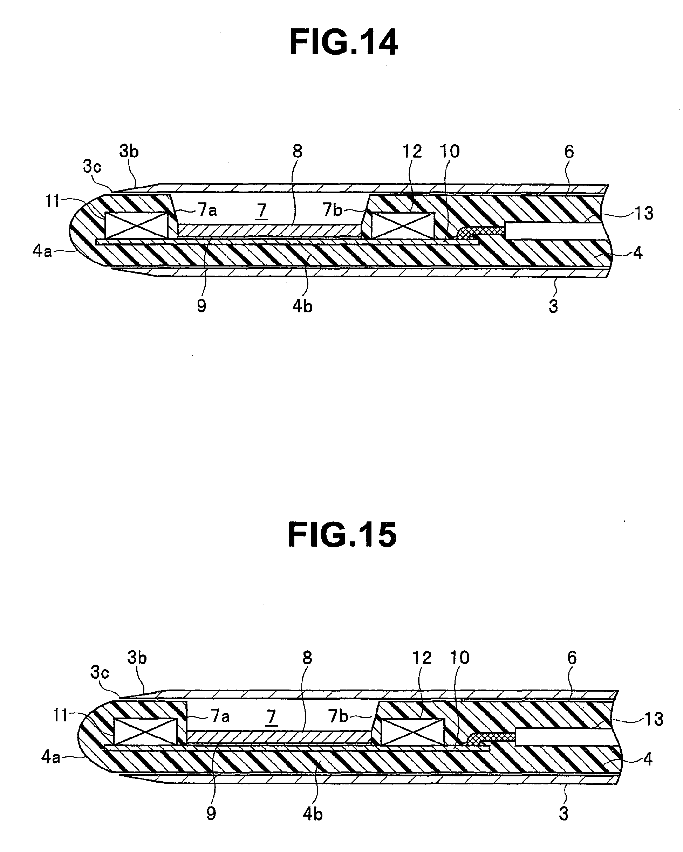

A third embodiment of the present invention is shown in Fig. 14. Note that components the same as the components in the first embodiment are denoted by the same reference numerals and signs and explanation of the components is omitted. In the present embodiment, both of the front portion wall surface 7a and the rear portion wall surface 7b of the recessed portion for specimen sampling 7 are formed as slopes expanding from a bottom surface side on which the capacitive ultrasound transducer array 8 is fixedly provided to an outer circumferential direction.

Ultrasound emitted from the capacitive ultrasound transducer array 8 fixedly provided in the bottom portion of the recessed portion for specimen sampling 7 has a side lobe having low sound pressure emitted in a direction deviating from the center axis. When the front portion wall surface 7a and the rear portion wall surface 7b are vertically erected, the side lobe tends to be reflected. When a reflected wave of the side lobe interferes with a reflected wave of the original ultrasound (a main lobe), a side lobe artifact occurs.

In the present embodiment, the front portion wall surface 7a and the rear portion wall surface 7b are formed as the slopes expanding from a bottom surface side on which the capacitive ultrasound transducer array 8 is fixedly provided to the outer circumferential direction. Therefore, the side lobe is less easily reflected from the respective wall surfaces 7a and 7b. Consequently, it is possible to suppress the occurrence of the side lobe artifact.

### [Fourth Embodiment]

A fourth embodiment of the present invention is shown in Fig. 15. The present embodiment is a modification of the third embodiment described above. In the third embodiment, both of the front portion wall surface 7a and the rear portion wall surface 7b are formed as the slopes. However, in the present embodiment, only the rear portion wall surface 7b is formed as a slope the same as the slope in the third embodiment.

When the front portion wall surface 7a is inclined as in the third embodiment described above, the occurrence of the side lobe artifact can be reduced. However, since an angle formed by the front portion wall surface 7a and the outer circumference is an obtuse angle, cutting performance (shearing force) at the time when the distal end edge 3c of the flexible sheath 3 is extruded crossing the front portion wall surface 7a is deteriorated.

In the present embodiment, since the rear portion wall surface 7b is formed as the inclined surface, it is possible to reduce the occurrence of the side lobe artifact. On the other hand, since the front portion wall surface 7a is vertically erected, it is possible to secure cutting performance (shearing force) at the time when the distal end edge 3c of the flexible sheath 3 is extruded crossing the front portion wall surface 7a.

### [Fifth Embodiment]

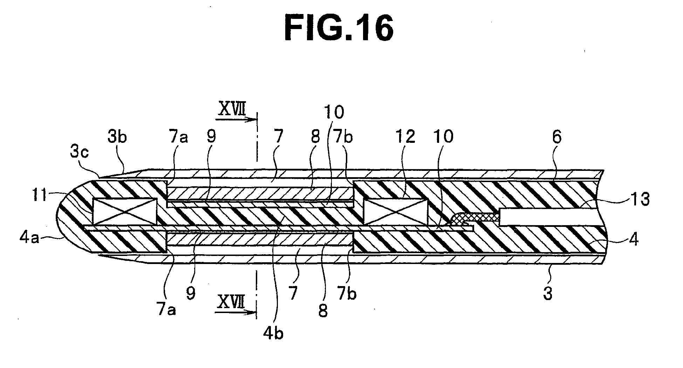

A fifth embodiment of the present invention is shown in Figs. 16 to 20. In the present embodiment, a plurality of the capacitive ultrasound transducer arrays 8 are disposed in the recessed portion for specimen sampling 7. Note that components the same as the components in the first embodiment are denoted by the same reference numerals and signs and explanation of the components is omitted.

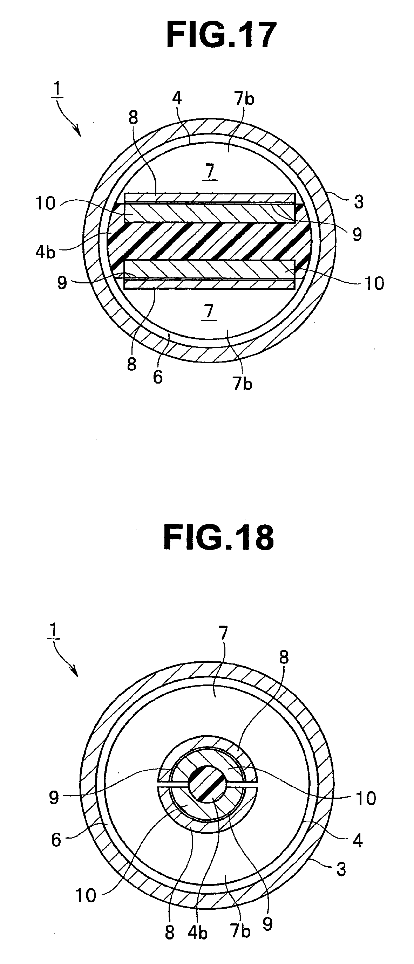

In an implementation aspect shown in Figs. 16 and 17, recessed portions for specimen sampling 7 are formed on both sides across the base portion 4b in the center. The capacitive ultrasound transducer arrays 8 are disposed on both surfaces of the base portion 4b, which are bottom surfaces of the recessed portions for specimen sampling 7. Note that flexible printed boards 10 mounted with the respective capacitive ultrasound transducer arrays 8 via backing materials 9 are illustrated as independent from each other in Fig. 17. However, actually, the flexible printed boards 10 are formed by one board.

In this implementation aspect, two recessed portions for specimen sampling 7 are fixedly provided on both the surfaces across the base portion 4b. Therefore, it is possible to more surely sample the specimen 14a from a target region of the body interior 14.

In an implementation aspect shown in Fig. 18, the recessed portion for specimen sampling 7 is formed in a toric shape centering on the base portion 4b, two capacitive ultrasound transducer arrays 8 are formed in an arcuate shape in section attachable to an outer circumference of the base portion 4b. The respective capacitive ultrasound transducer arrays 8 are mounted on one flexible printed board 10 via respective backing materials 9.

According to this implementation aspect, the capacitive ultrasound transducer array 8 is disposed around the base portion 4b formed in the axis of the insertion portion 4. Therefore, it is possible to perform radial electron scanning around the axis without rotating the insertion portion 4 and more accurately detect a target region of the body interior 14. In addition, since the recessed portion for specimen sampling 7 is formed in the toric shape, when the target region is constricted, the recessed portion for specimen sampling 7 is opened to easily capture a lesion portion or the like of the target region into the recessed portion for specimen sampling 7. Therefore, it is possible to easily sample the specimen 14a from the target region.

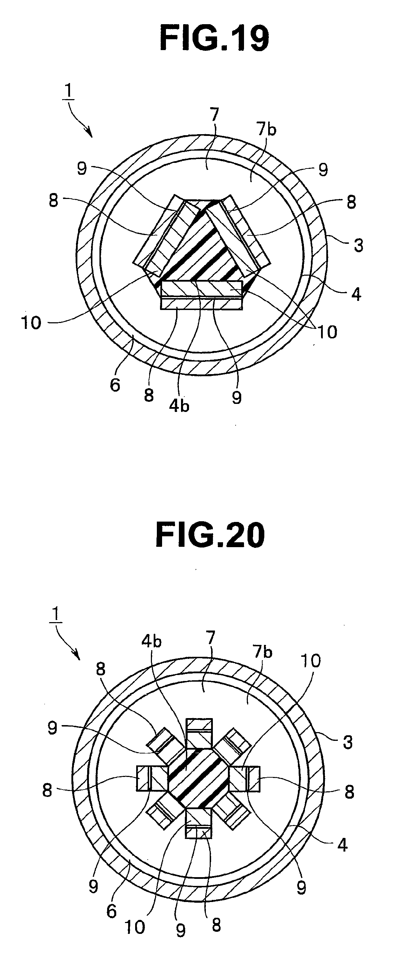

In an implementation aspect shown in Fig. 19, the base portion 4b is formed in a triangular shape in section in the axis of the insertion portion 4. The recessed portion for specimen sampling 7 is formed around the base portion 4b. One flexible printed board 10 is disposed on three surfaces of the base portion 4b. Three capacitive ultrasound transducer arrays 8 are respectively mounted on respective surfaces of the flexible printed board 10 via the backing materials 9.

According to this implementation aspect, the body interior 14 can be subjected to the ultrasound scanning by the respective capacitive ultrasound transducer arrays 8 provided on the three surfaces of the base portion 4b. Therefore, compared with the implementation aspects shown in Figs. 17 and 18, it is possible to more quickly specify the region to be the specimen 14a such as a lesion portion of the target region.

In an implementation aspect shown in Fig. 20, the base portion 4b is formed in an octagonal shape in section in the axis of the insertion portion 4. The recessed portion for specimen sampling 7 is formed around the base portion 4b. One flexible printed board 10 is disposed on eight surfaces of the base portion 4b. Eight capacitive ultrasound transducer arrays 8 are respectively mounted on respective surfaces of the flexible printed board 10 via the backing material 9.

According to this implementation aspect, ultrasound scanning, which is similar to radial electron scanning, can be performed in the body interior 14 by the respective capacitive ultrasound transducer arrays 8 provided on the eight surfaces of the base portion 4b. Therefore, it is possible to specify the region to be the specimen 14a and more highly accurately detect whether or not the region to be the specimen 14a is captured into the recessed portion for specimen sampling 7.

Incidentally, the capacitive ultrasound transducer arrays 8 provided in the biopsy treatment instrument 1 according to the present embodiment are not for specifying the target region in the body interior 14 but only for specifying the region to possibly be the specimen 14a such as a lesion portion of the target region and further determining whether or not the region is captured into the recessed portion for specimen sampling 7. Therefore, an accurate ultrasound scanning result is not requested and relatively rough ultrasound scanning may be performed. Therefore, two or three capacitive ultrasound transducer arrays 8 disposed around the base portion 4b can sufficiently function. Further, it is possible to perform more accurate ultrasound scanning, which is similar to radial electronic scanning, by disposing eight capacitive ultrasound transducer arrays 8 around the base portion 4b.

### [Sixth Embodiment]

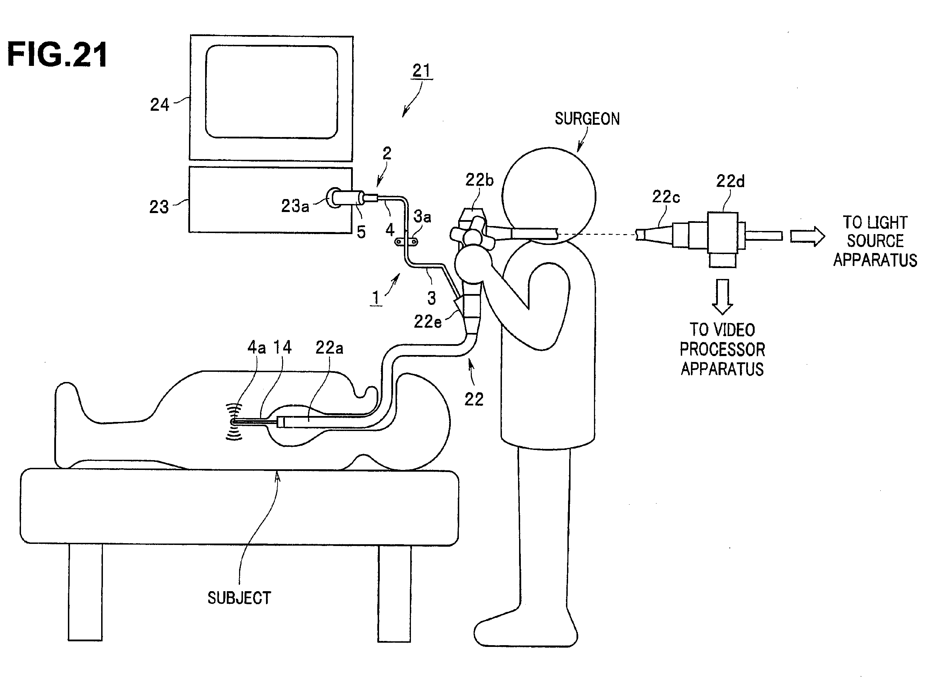

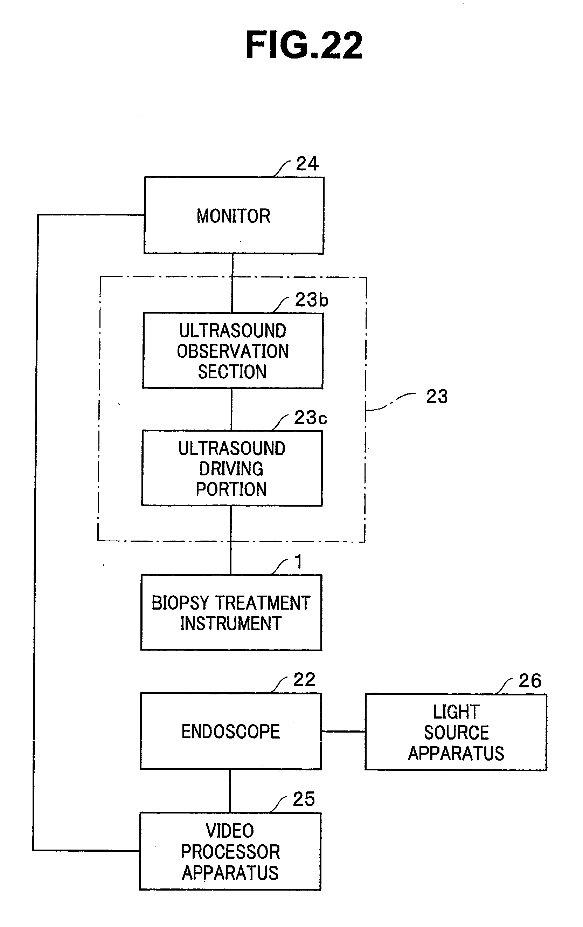

A sixth embodiment is shown in Figs. 21 and 22. In the figures, an endoscope system 21 including any one of the biopsy treatment instruments 1 explained in the first to fifth embodiments described above is shown.

An endoscope 22 included in the endoscope system 21 includes an elongated endoscope insertion portion 22a having flexibility. An operation section 22b is provided on a side of a surgeon's hand of the endoscope insertion portion 22a. Further, a universal cord 22c is extended from the operation section 22b. A scope connector 22d is provided at an end portion of the universal cord 22c. A video processor apparatus 25 and a light source apparatus 26 are connected to the scope connector 22d (see Fig. 22).

A treatment instrument insertion port 22e is opened near a coupling portion of the endoscope insertion portion 22a and the operation section 22b. A rear end of a treatment instrument channel (not shown) communicates with the treatment instrument insertion port 22e. The treatment instrument channel is formed in the endoscope insertion portion 22a. A distal end of the treatment instrument channel is opened in a distal end face of the endoscope insertion portion 22a.

On the other hand, the connector portion 5 provided at a rear end of the treatment instrument main body 2 provided in the biopsy treatment instrument 1 is connected to a connector receiving portion 23a of an ultrasound observation apparatus 23. As shown in Fig. 22, the ultrasound observation apparatus 23 includes an ultrasound observation section 23b and an ultrasound driving portion 23c. The ultrasound driving portion 23c generates an ultrasound driving signal for driving the capacitive ultrasound transducer array 8, which is provided in the treatment instrument main body 2 of the biopsy treatment instrument 1, via a multiplexer. The ultrasound observation section 23b converts a reflected wave received by the capacitive ultrasound transducer array 8 or a waveform of the reflected waveform into a physical quantity and causes a monitor 24 to display the physical quantity.

When the surgeon samples the specimen 14a from a target region of a subject, firstly, the surgeon inserts the endoscope insertion portion 22a of the endoscope 22 from an oral cavity of the subject or the like and leads the endoscope insertion portion 22a to near the target region while observing an endoscope image. Subsequently, the surgeon inserts, from the treatment instrument insertion port 22e of the endoscope 22, both of the insertion portion 4 provided in the treatment instrument main body 2 of the biopsy treatment instrument 1 and the flexible sheath 3 sheathed over the insertion portion 4. The surgeon projects the insertion portion 4 and the flexible sheath 3 from a distal end of the endoscope insertion portion 22a through the treatment instrument channel communicating with the treatment instrument insertion port 22e, inserts the insertion portion 4 and the flexible sheath 3 into the body interior 14 linked to the target region, and performs the operation explained in the first to fifth embodiments described above to sample the specimen 14a of the target region.

As explained above, according to the present embodiment, since any one of the biopsy treatment instruments 1 according to the first to fifth embodiments is provided in the endoscope system 21, it is possible to surely sample the specimen 14a from the subject by inserting the endoscope once.

## Claims

1. A biopsy treatment instrument (1) comprising:
a tubular portion (3);
a bar-like portion (4) capable of advancing and retracting in the tubular portion (3);
a recessed portion (7) provided on a side surface of the bar-like portion (4); and
an ultrasound observation section (8) provided on a bottom surface of the recessed portion (7);
**characterized in that** the instrument (1) further comprises:
a slide portion (6) that relatively slides the bar-like portion (4) and the tubular portion (3) so that the recessed portion (7) opens and closes, wherein
an outer surface (3b) of a distal end of the tubular portion (3) is formed in a single-edge shape inclining toward a tangential direction with respect to a distal end face (4a) of the bar-like portion (4).

2. The biopsy treatment instrument (1) according to claim 1, wherein
the distal end face (4a) of the bar-like portion (4) is formed in a rotating body shape of a conical curve centering on an axis of the bar-like portion (4),.

3. The biopsy treatment instrument (1) according to claim 1, wherein a circuit element (11, 12) that processes an ultrasound signal transmitted and received by the ultrasound observation section (8) is incorporated in a distal end face (4a) of the bar-like portion (4).

4. The biopsy treatment instrument (1) according to claim 1, wherein a wall surface (7a, 7b) of the recessed portion (7) is expanded from a bottom surface of the recessed portion (7) to an outer circumferential direction.

5. The biopsy treatment instrument (1) according to claim 1, wherein the bar-like portion(4) includes a suction conduit (15) opened in the recessed portion (7).

6. The biopsy treatment instrument (1) according to claim 1, wherein the recessed portion (7) is arranged in an entire circumference of the bar-like portion (4).

7. The biopsy treatment instrument (1) according to claim 1, wherein the ultrasound observation section (8) is a capacitive ultrasound transducer manufactured using a micro machine manufacturing process.

## Patentansprüche

1. Biopsiebehandlungsinstrument (1) aufweisend:
ein röhrenförmiges Teil (3);
ein stabartiges Teil (4), das dazu in der Lage ist, in dem röhrenförmigen Teil (3) vor und zurück bewegt zu werden;
ein ausgespartes Teil (7), dass an einer seitlichen Fläche des stabartigen Teils (4) bereitgestellt ist; und
einen Ultraschallbeobachtungsabschnitt (8), der an einer unteren Fläche des ausgesparten Teils (7) bereitgestellt ist;
**dadurch gekennzeichnet, dass** das Instrument (1) ferner aufweist:
ein Schiebeteil (6), das das stabartige Teil (4) und das röhrenförmige Teil (3) relativ zueinander schiebt, so dass sich das ausgesparte Teil (7) öffnet und schließt, wobei
eine äußere Fläche (3b) eines distalen Endes des röhrenförmigen Teils (3) in einer einkantigen Form gebildet ist, die sich hinsichtlich einer distalen Endfläche (4a) des stabartigen Teils (4) zu einer Tangentialrichtung neigt.

2. Biopsiebehandlungsinstrument (1) nach Anspruch 1, bei dem
die distale Endfläche (4a) des stabartigen Teils (4) in einer Drehkörperform einer konischen Kurve gebildet ist, die auf einer Achse des stabartigen Teils (4) zentriert ist.

3. Biopsiebehandlungsinstrument (1) nach Anspruch 1, bei dem in einer distalen Endfläche (4a) des stabartigen Teils (4) ein Schaltungselement (11, 12) enthalten ist, das ein von dem Ultraschallbeobachtungsabschnitt (8) gesendetes und empfangenes Ultraschallsignal verarbeitet.

4. Biopsiebehandlungsinstrument (1) nach Anspruch 1, bei dem sich eine Wandfläche (7a, 7b) des ausgesparten Teils (7) von einer unteren Fläche des ausgesparten Teils (7) zu einer äußeren Umfangsrichtung ausweitet.

5. Biopsiebehandlungsinstrument (1) nach Anspruch 1, bei dem das stabartige Teil (4) einen Saugkanal (15) enthält, der sich in dem ausgesparten Teil (7) öffnet.

6. Biopsiebehandlungsinstrument (1) nach Anspruch 1, bei dem das ausgesparte Teil (7) in einem gesamten Umfang des stabartigen Teils (4) angeordnet ist.

7. Biopsiebehandlungsinstrument (1) nach Anspruch 1, bei dem der Ultraschallbeobachtungsabschnitt (8) ein kapazitiver Ultraschallwandler ist, der unter Verwendung eines Mikrobearbeitungsherstellungsprozesses hergestellt wurde.

## Revendications

1. Instrument (1) de traitement de biopsie comprenant :
une partie (3) tubulaire ;
une partie (4) en forme de barre capable d'avancer et de reculer dans la partie (3) tubulaire ;
une partie (7) évidée prévue sur une surface latérale de la partie (4) en forme de barre ; et
une section (8) d'observation par ultrasons prévue sur une surface de fond de la partie (7) évidée ;
**caractérisé en ce que** l'instrument (1) comprend en outre :
une partie (6) coulissante qui fait coulisser de manière relative la partie (4) en forme de barre et la partie (3) tubulaire de sorte que la partie (7) évidée s'ouvre et se ferme, dans lequel
une surface externe (3b) d'une extrémité distale de la partie (3) tubulaire est formée à une forme à bord unique s'inclinant vers une direction tangentielle par rapport à une face (4a) d'extrémité distale de la partie (4) en forme de barre.

2. Instrument (1) de traitement de biopsie selon la revendication 1, dans lequel
la face (4a) d'extrémité distale de la partie (4) en forme de barre est formée à une forme de corps rotatif d'une courbe conique se centrant sur un axe de la partie (4) en forme de barre.

3. Instrument (1) de traitement de biopsie selon la revendication 1, dans lequel un élément (11, 12) de circuit qui traite un signal ultrasonore transmis et reçu par la section (8) d'observation par ultrasons est incorporé dans une face (4a) d'extrémité distale de la partie (4) en forme de barre.

4. Instrument (1) de traitement de biopsie selon la revendication 1, dans lequel une surface de paroi (7a, 7b) de la partie (7) évidée est étendue à partir d'une surface de fond de la partie (7) évidée vers une direction circonférentielle externe.

5. Instrument (1) de traitement de biopsie selon la revendication 1, dans lequel la partie (4) en forme de barre comprend un conduit (15) d'aspiration ouvert dans la partie (7) évidée.

6. Instrument (1) de traitement de biopsie selon la revendication 1, dans lequel la partie (7) évidée est disposée dans une circonférence entière de la partie (4) en forme de barre.

7. Instrument (1) de traitement de biopsie selon la revendication 1, dans lequel la section (8) d'observation par ultrasons est un transducteur ultrasonore capacitif fabriqué en utilisant un processus de fabrication par micro-usinage.
